# EUROPEAN PATENT APPLICATION

(11) **EP 4 552 618 A1**
(43) Date of publication of application: **14.05.2025**
(21) Application number: 23845494.6
(22) Date of filing: 24.07.2023
(51) Int. Cl.: A61F 2/82

(54) **VASCULAR STENT AND PREPARATION METHOD THEREFOR**

(30) Priority: 29.07.2022 CN 202210911128
(71) Applicant: Shanghai MicroPort Medical (Group) Co., Ltd., Shanghai 201203 (CN)
(72) Inventor: GU, Jialei, Shanghai 201203 (CN); LIU, Wei, Shanghai 201203 (CN); WANG, Xueqin, Shanghai 201203 (CN); YUE, Bin, Shanghai 201203 (CN)
(74) Representative: Marks & Clerk LLP
(86) International application number: PCT/CN2023/108859
(87) International publication number: WO 2024/022284

(57) **Abstract**

A vascular stent and a preparation method therefor are provided. The vascular stent includes a cylindrical stent and a restricting member (200). The cylindrical stent includes at least one flexible sheet (100). A circumferential direction of the cylindrical stent is defined as a winding direction. The at least one flexible sheet (100) is wound along the winding direction. The restricting member (200) is configured to restrict the flexible sheet (100) from being wound along a direction that would cause a decrease of the diameter of the cylindrical stent.

## Description

### CROSS-REFERENCE TO RELATED APPLICATIONS

The present application claims priority to Chinese patent application No. 2022109111288, filed on July 29, 2022, and entitled "VASCULAR STENT AND PREPARATION METHOD THEREFOR", the entire content of which is incorporated herein by reference.

### TECHNICAL FIELD

The present application relates to the field of medical device technologies, and in particular, to a vascular stent and a preparation method therefor.

### BACKGROUND

Biodegradable vascular stents can be degraded by the human body after assisting in vascular remodeling. They not only do not affect the vascular elasticity after surgery, but also create a possibility for potential stent intervention for recurrent vascular stenosis in the future.

At present, the mainstream biodegradable vascular stents are made of degradable polymers, such as L-polylactic acid. However, since the mechanical properties of the polymer itself are not as good as those of conventional metal materials, the existing biodegradable vascular stents often have weak support force, or are designed with thicker stent beams to have a support force similar to that of metal stents. Therefore, how to improve the support force of biodegradable vascular stents is an urgent problem to be solved in further research and development of biodegradable vascular stents.

### SUMMARY

A vascular stent and a preparation method therefor are provided in the present application according to various embodiments of the present application.

A vascular stent is provided in the present application. The vascular stent includes: a cylindrical stent and a restricting member. The cylindrical stent includes at least one flexible sheet, a circumferential direction of the cylindrical stent is defined as a winding direction, the at least one flexible sheet is wound along the winding direction. The restricting member is provided on the flexible sheet, and is configured to restrict the flexible sheet from being wound along a direction that would cause a decrease of the diameter of the cylindrical stent.

In an embodiment, the restricting member and the flexible sheet are integrally formed.

In an embodiment, the restricting member is fixedly connected to the flexible sheet.

In an embodiment, the flexible sheet has overlapping areas, the overlapping areas of the flexible sheet form interlayer contact within the cylindrical stent, and the restricting member is at least partially located in the overlapping areas.

In an embodiment, the cylindrical stent is formed by winding a single flexible sheet, the overlapping areas of the single flexible sheet include different parts, and the different parts form interlayer contact within the cylindrical stent.

In an embodiment, the cylindrical stent is formed by winding at least two flexible sheets, and the overlapping areas on different flexible sheets form interlayer contact within the cylindrical stent.

In an embodiment, each of the flexible sheets is wound to form a cylindrical unit, the cylindrical unit has a side opening, and the at least two flexible sheets are stacked to form the cylindrical stent.

In an embodiment, the at least two side openings are evenly distributed along the circumferential direction of the cylindrical stent.

In an embodiment, the restricting member comprises unidirectional limiting racks arranged in the overlapping areas, and the unidirectional limiting racks within adjacent overlapping areas which form interlayer contact slide relative to each other or lock each other in two opposite circumferential directions, respectively.

In an embodiment, the restricting member comprises unidirectional limiting fluff arranged on the overlapping area, and the unidirectional limiting fluff within adjacent overlapping areas which form interlayer contact generates a first friction force and a second friction force respectively along two opposite circumferential directions, and the first friction force and the second friction force are different.

In an embodiment, the cylindrical stent defines a circumferential restricting trajectory around the circumferential direction of the cylindrical stent, and the restricting member restricts the relative slidably winding between adjacent overlapping areas of the flexible sheet that form interlayer contact, along the circumferential restricting trajectory.

In an embodiment, the flexible sheet is provided with transition ramps at ends thereof along the circumferential direction of the cylindrical stent, and the cylindrical stent forms a smooth surface through the transition ramps.

In an embodiment, the flexible sheet is made of a degradable polymer material.

In an embodiment, the restricting member comprises multiple restricting members, and the multiple restricting members are distributed on the flexible sheet along an axial direction of the cylindrical stent.

In an embodiment, a length of the restricting member along the circumferential direction of the cylindrical stent is a first circumferential length, a length of the flexible sheet along the circumferential direction of the cylindrical stent is a second circumferential length, and the first circumferential length is equal to the second circumferential length.

In an embodiment, a length of the restricting member along the circumferential direction of the cylindrical stent is a first circumferential length, a length of the flexible sheet along the circumferential direction of the cylindrical stent is a second circumferential length, the first circumferential length is smaller than the second circumferential length, and the multiple restricting members are staggered along the axial direction of the cylindrical stent.

A preparation method for a vascular stent is provided in the present application. The method includes the following steps: providing a restricting member on the flexible sheet, winding and shaping the flexible sheet into a cylindrical stent, and using the restricting member to restrict the flexible sheet from being wound along a direction that would cause a decrease of the diameter of the cylindrical stent.

The details of one or more embodiments of the present application are set forth in the accompanying drawings and the description below, and other features, objects, and advantages of the present application will be apparent from the description, the drawings, and the claims.

### BRIEF DESCRIPTION OF THE DRAWINGS

In order to describe the technical solutions in the embodiments of the present application or the conventional technology more clearly, the following will briefly introduce the accompanying drawings required for describing the embodiments or the conventional technology. Apparently, the accompanying drawings in the following description are merely embodiments of the present application, and for a person of ordinary skill in the art, other drawings can be obtained based on the disclosed drawings without creative efforts.
FIG. 1 is a schematic diagram of a vascular stent formed by winding a single flexible sheet provided in some embodiments of the present application.
FIG. 2 is a schematic diagram of a structure of the vascular stent shown in FIG. 1, with an increased diameter.
FIG3 is a schematic diagram of a three-dimensional structure of the vascular stent shown in FIG. 1.
FIG. 4 is a schematic diagram of a vascular stent formed by winding a plurality of flexible sheets provided in some embodiments of the present application.
FIG. 5 is a schematic diagram of a structure of a vascular stent shown in FIG. 4, with an increased diameter.
FIG. 6 is a schematic diagram of a structure of a restricting member provided in some embodiments of the present application.
FIG. 7 is a schematic diagram of a structure of the restricting member shown in FIG. 6 in a locking state.
FIG. 8 is a schematic diagram of a structure of a restricting member provided in some other embodiments of the present application.
FIG. 9 is a schematic diagram of a structure of the restricting member shown in FIG. 8 in a locking state.
FIG. 10 is a schematic diagram showing a distribution of restricting members on a flexible sheet provided in some embodiments of the present application.
FIG. 11 is a schematic diagram showing a distribution of restricting members on a flexible sheet provided in some other embodiments of the present application.

Description of reference signs of the accompanying drawings:
100-Flexible sheet; 200-Restricting member;
110-Overlapping area; 120-Adjacent overlapping areas; 130-Side opening; 140-Transition ramp.

### DETAILED DESCRIPTION OF THE EMBODIMENTS

The technical solutions in the embodiments of the present application will be clearly and completely described with reference to the accompanying drawings. Apparently, the described embodiments are only some but not all of the embodiments of the present application. Based on the embodiments of the present application, all other embodiments obtained by a person of ordinary skill in the art without creative efforts shall fall within the protection scope of the present application.

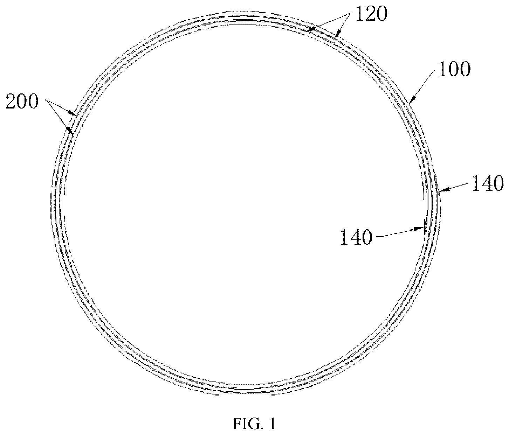

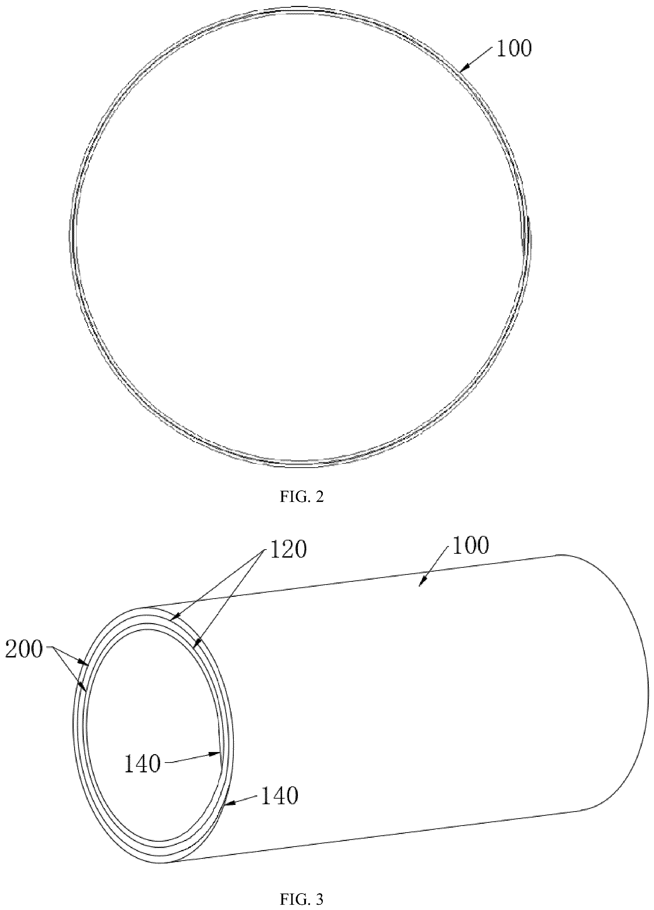

Referring to FIG. 1 to FIG.11, a vascular stent is provided in an embodiment of the present application. The vascular stent includes a cylindrical stent. The cylindrical stent includes at least one flexible sheet 100. A circumferential direction of the cylindrical stent is defined as a winding direction. The at least one flexible sheet 200 is wound along the winding direction. The vascular stent further includes a restricting member 200. The restricting member 200 is provided on the flexible sheet 100, and configured to restrict the flexible sheet 100 from being wound along a direction that would cause a decrease of the diameter of the cylindrical stent.

It should be noted that the circumferential direction of the cylindrical stent includes two opposite directions, for example, a clockwise direction and a counterclockwise direction shown in FIG. 1. In the winding state shown in FIG. 1, winding in the clockwise direction will increase the diameter of the cylindrical stent, and winding in the counterclockwise direction will decrease the diameter of the cylindrical stent. Those skilled in the art can understand from FIG. 1 that the restricting member 200 restricts the flexible sheet 100 from being wound along a direction that would cause a decrease of the diameter of the cylindrical stent. The other embodiments are the same and will not be repeated here.

It should be noted that the flexible sheet 100, when wound to form the cylindrical stent, is movable. That's to say, although the flexible sheet 100 forms a cylindrical stent with a relatively stable structure, adjacent layers (surfaces) of the flexible sheet 100 that form interlayer contact maintain an ability to slide relative to each other, rather than completely fixing the winding state of the cylindrical stent. Therefore, when an external force is applied to the cylindrical stent, for example, a force is applied from the inside to the outside along a radial direction of the cylindrical stent, the flexible sheet 100 forming the cylindrical stent can continue to be wound under the external force, thereby increasing the diameter of the cylindrical stent.

Therefore, when the restricting member 200 is provided on the flexible sheet 100, the restricting member 200 can be used to actively restrict the movable winding of the flexible sheet. The restricting member 200 can be used to restrict the contraction winding of the flexible sheet 100, and the contraction winding refers to that the flexible sheet 100 can reduce the diameter of the cylindrical stent by winding. At the same time, the restricting member 200 does not restrict or hardly restrict the expansive winding of the flexible sheet 100, and the expansive winding refers to that the flexible sheet 100 can increase the diameter of the cylindrical stent by winding. The restricting member 200 can be provided on the flexible sheet 100 in various forms, such as the restricting member 200 being integrally formed with the flexible sheet 100, the restricting member 200 being fixedly connected to the flexible sheet 100, or the restricting member 200 being connected to the flexible sheet 100 in separable manners such as bonding, clamping, etc., which are not limited here.

Therefore, after the restricting member 200 is used to form a unidirectional restriction on the movable winding of the flexible sheet 100, the cylindrical stent (equivalent to the vascular stent) can be delivered to a target position of a blood vessel in a winding form with a smaller diameter. When the cylindrical stent reaches the target position of the blood vessel, the operator can use a balloon or other component that can achieve automatic expansion or contraction to apply an external force to the flexible sheet 100 forming the cylindrical stent inside the cylindrical stent, which can cause the flexible sheet 100 to be expansively wound, so that the flexible sheet 100 can increase the diameter of the cylindrical stent by expansive winding, and the cylindrical stent can be stably attached to an inner wall of the blood vessel.

After the diameter of the cylindrical stent is e increased, the cylindrical stent will form a tight fit with the inner wall of the blood vessel. Although the inner wall of the blood vessel will exert a reaction force on the cylindrical stent in an opposite direction at this time, the cylindrical stent will still maintain its present diameter with the help of the restriction of the restricting member 200, rather than reduce its diameter due to the reaction force exerted by the inner wall of the blood vessel. Obviously, the restriction of the restricting member 200 enables the cylindrical stent (equivalent to the vascular stent) to have a stronger supporting force and maintain a stable and tight fit with the inner wall of the blood vessel in the blood vessel, and can also enable the cylindrical stent (equivalent to the vascular stent) to have a higher fatigue resistance.

The restricting direction of the restricting member 200 for the flexible sheet 100 can be predetermined. For example, a standard circumferential restricting trajectory can be set so that the restricting member 200 restricts the relative slidably winding between adjacent overlapping areas 120 of the flexible sheet 100 that form interlayer contact, along the circumferential restricting trajectory. The circumferential restricting trajectory can be defined as being perpendicular to an axial direction of the cylindrical stent. Therefore, the restricting function of the restricting member 200 along the circumferential restricting trajectory can ensure that the flexible sheet 100 is only wound along the circumferential direction, and then the cylindrical stent formed by the winding produces structural changes only in the radial direction, such as radial expansion, without affecting the generation of the structural changes in the axial direction, such as changes in the length of the cylindrical stent, i.e., the length of the cylindrical stent will not be changed in the axial direction due to winding misalignment.

The function of the restricting member 200 is to restrict the flexible sheet 100 from being wound along a direction that would cause a decrease of the diameter of the cylindrical stent. Therefore, as long as the restricting function can be achieved, the restricting member 200 can adopt any structural form. For example, the restricting member 200 can be assembled with the flexible sheet 100 in a separable manner, or the restricting member 200 can also be integrated into the flexible sheet 100 to form a unified structure. The restricting member 200 provided on the flexible sheet 100 can be any form such as a groove structure, a protrusion structure, a rack structure, a fluff structure, etc. Those skilled in the art can select a suitable structural form according to needs, without limitation herein.

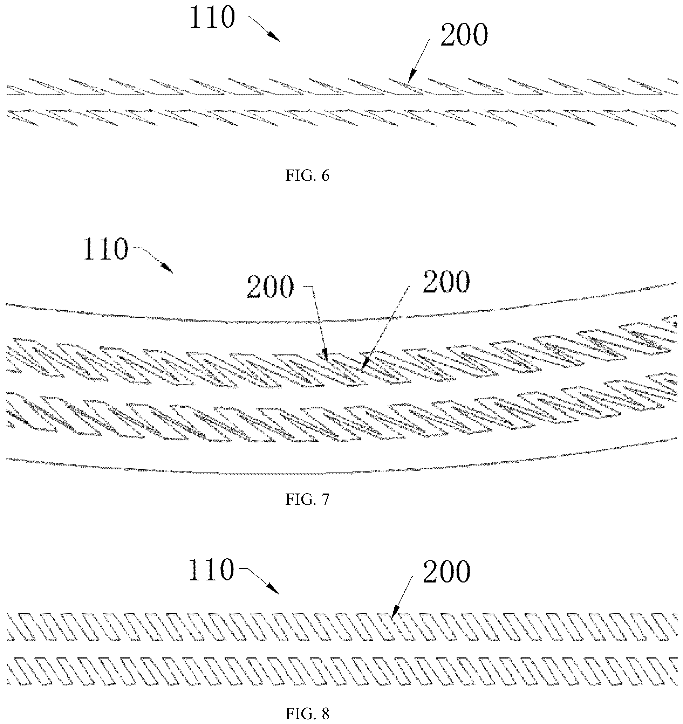

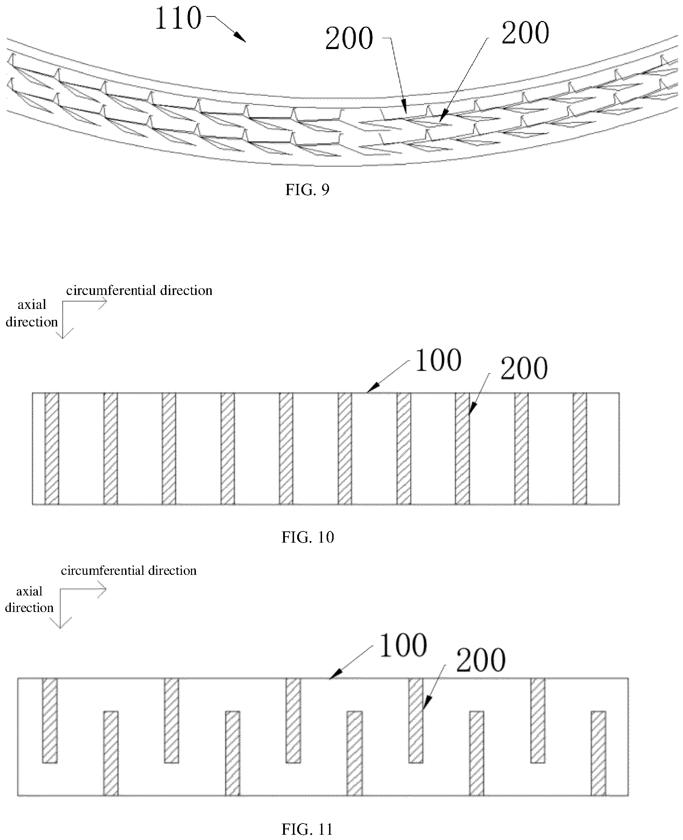

Referring to FIG. 6 to FIG. 9, in some embodiments, the restricting member 200 may include unidirectional limiting racks arranged on the overlapping area 110. As shown in FIG. 6 and FIG. 7, when teeth of the unidirectional limiting racks are spike-shaped, the entire unidirectional limiting racks can be formed by connecting a large number of microneedles along the circumferential direction. The lengths of the microneedles can be 10 microns to 50 microns, and the inclination angles of the microneedles can be 20° to 90°. An absorbable solid microneedle preparation process can be selected as the preparation process of the microneedles, such as LIGA (electroforming technology) and micro injection molding, etc. Alternatively, as shown in FIG. 8 and FIG. 9, when the teeth of the unidirectional limiting racks are long strips, the entire unidirectional limiting rack can be formed by connecting a large number of rectangular racks along the circumferential direction, and the formed unidirectional limiting rack is similar to a fishbone shape. The rectangular racks can be formed by cutting, and a polymer cutting process can be used, such as laser milling, or the rectangular racks can be directly formed in a suitable mold.

Therefore, the unidirectional limiting racks within adjacent overlapping areas 120 which form interlayer contact can slide relative to each other or lock each other along two opposite circumferential directions of the cylindrical stent respectively. Continuing to refer to FIG. 7, when the unidirectional limiting racks are formed by connecting a large number of microneedles along the circumferential direction, the microneedles between adjacent unidirectional limiting racks can be hooked together so that they can only slide relative to each other in one direction and lock each other in the other direction. Alternatively, referring to FIG. 9, when the unidirectional limiting racks are formed by connecting a large number of rectangular racks along the circumferential direction, the rectangular racks of the unidirectional limiting racks can fit together on an inner curved side to form a step structure, and can open on an outer curved side to form a claw structure. Further, the claw structure can be used to form a relative buckle. Since the step structure and the claw structure are both unidirectional, they can form a forward structural sliding between adjacent layers and a reverse structural locking between adjacent layers.

In some embodiments, the restricting member 200 may also include unidirectional limiting fluff arranged on the overlapping area 110. The unidirectional limiting fluff can be mainly used in electrostatically spun polymer sheets or films. For example, the unidirectional restricting fluff can form bristles, grasping bristles or grinding bristles through a fabric raising process, and electrostatically spun fibers are raised unidirectionally in the same direction. If a relative movement direction between adjacent overlapping areas 120 of the flexible sheet 100 is the same as a fluff raising direction, a friction between adjacent overlapping areas 120 is low, so that the adjacent overlapping areas 120 can slide smoothly. If a sliding direction is opposite to the fluff raising direction, the friction between adjacent overlapping areas 120 of the flexible sheet 100 increases sharply, and a sliding movement between adjacent overlapping areas 120 is restricted, resulting in a significantly increased supporting force of the cylindrical stent. The unidirectional limiting fluff within adjacent overlapping areas 120 which form interlayer contact is capable of generating a first friction force and a second friction force respectively along two opposite circumferential directions of the cylindrical stent. The first friction force and the second friction force are different. Those skilled in the art can adjust a raising state of the fluff according to needs, adjust a difference between the first friction force and the second friction force, and further adjust the smoothness and restriction when sliding along different directions between adjacent overlapping areas 120.

The restricting member 200 is provided on the flexible sheet 100, which can be understood as the restricting member 200 being located within an area of the flexible sheet 100 itself, or it can be understood as the restricting member 200 being located outside an area of the flexible sheet 100 itself, and simply forming a connection with the flexible sheet 100.

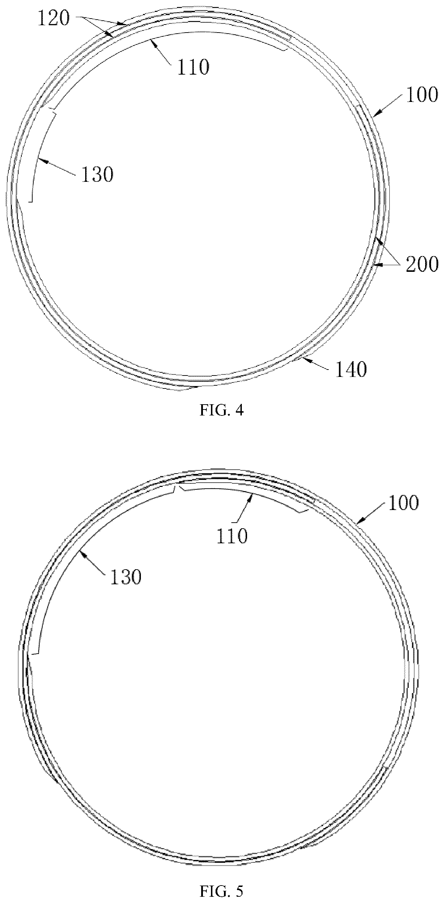

For example, when the restricting member 200 is located within the area of the flexible sheet 100 itself, the flexible sheet 100 needs to be wound into a closed cylinder to form the cylindrical stent, and then the restricting function is performed with the help of the restricting member 200 in the area of the flexible sheet 100 itself. At this time, at least a part of the area of the flexible sheet 100 is defined as the overlapping area 110. Referring to FIG. 4 and FIG. 5, the flexible sheet 100 has an overlapping area 110. The overlapping area 110 of the flexible sheet 100 is capable of forming interlayer contact within the cylindrical stent, and the restricting member can also be at least partially located within the overlapping area 110. When the overlapping area 110 of the flexible sheet 100 form interlayer contact by winding, the adjacent overlapping areas 120 which form interlayer contact in the flexible sheet 100 can use the restricting member 200 to form mutual restriction between each other.

For another example, when the restricting member 200 is located outside the area of the flexible sheet 100 itself and is simply connected with the flexible sheet 100, the restricting member 200 can be connected to an edge of the flexible sheet 100 and is completely located outside the area of the flexible sheet 100 itself. When the flexible sheet 100 is wound into a cylindrical stent, the restricting member 200 forms interlayer contact with a surface of the flexible sheet 100 based on the cylindrical structure formed by the winding of the flexible sheet 100, resulting in mutual restriction between the surface of the flexible sheet 100 and the restricting member 200 and thereby achieving the restricting effect.

It should be noted that interlayer contact means that when the flexible sheet 100 is wound to form a cylindrical stent, different surfaces of the flexible sheet 100 will form a stacked state. At this time, the contact between the different surfaces of the flexible sheet 100 is the interlayer contact of the flexible sheet 100. For example, as shown in FIG. 1 to FIG. 5, the adjacent overlapping areas 120 reflect the interlayer contact of different surfaces. The interlayer contact can be between the different surfaces of the same flexible sheet 100, or between different surfaces of different flexible sheets 100. This needs to be determined according to the flexible sheet 100 contained in the cylindrical stent and the winding method. Those skilled in the art can make reasonable selections according to needs, which are not specifically limited herein.

Alternatively, in some embodiments, when the restricting member 200 is located outside the area of the flexible sheet 100 itself and is simply connected with the flexible sheet 100, the flexible sheet 100 does not need to be wound into a closed cylinder to form the cylindrical stent. At this time, the restricting function of the restricting structure 200, located outside the area of the flexible sheet 100, can also be utilized to restrict the flexible sheet 100, which forms the cylindrical stent, from generating contraction winding that would reduce the diameter of the cylindrical stent, such that the cylindrical support maintains a non-closed structure with a fixed diameter.

The cylindrical stent can be formed by winding one or more flexible sheets 100. Different numbers of flexible sheets 100 used in the cylindrical stent lead to different winding forms of the flexible sheet 100. For example, in an embodiment, referring to FIG. 1 to FIG. 3, the number of the flexible sheet 100 is one, the cylindrical stent is formed by winding the one flexible sheet 100, and different parts within the overlapping area 110 of the flexible sheet 100 form interlayer contact within the cylindrical stent. In this winding method, the flexible sheet 100 is always wound in one circumferential direction. On a radial cross-section of the cylindrical stent formed by winding the flexible sheet 100, the winding structure of the flexible sheet 100 is in the form of a spiral structure, and the different parts within the overlapping area 110 represent different parts (or different small areas) distributed in the overlapping area 110.

Alternatively, in some embodiments, referring to FIG. 4 and FIG. 5, when the number of the flexible sheets 100 is at least two, the cylindrical stent is formed by winding the at least two flexible sheets 100, and the overlapping areas 110 on the different flexible sheets 100 form interlayer contact within the cylindrical stent. According to structural requirements of the cylindrical stent, the number of the flexible sheets 100 can be two, three, or four. Continuing to refer to FIG. 4 and FIG. 5, when the number of the flexible sheets 100 is three, the flexible sheets 100 each have overlapping areas 110 at both ends thereof in the circumferential direction. Depending on the interlayer contact of each flexible sheet 100 with other flexible sheets 100 during the winding process, the overlapping area 110 of the flexible sheet 100 can include a single surface or both surfaces of the flexible sheet 100. After the three flexible sheets 100 are wound along the circumferential direction, the overlapping areas 110 of adjacent flexible sheets 100 will form interlayer contact with each other, and then the three flexible sheets 100 will form a cylindrical stent by winding in the circumferential direction.

In the case where a plurality of flexible sheets 100 are wound to form the cylindrical stent, each of the flexible sheets 100 can be wound to form a cylindrical unit with a side opening 130. that's to say, the side opening 130 is intended for the single flexible sheet 100, and can be displayed before the flexible sheet 100 is wound into the cylindrical stent. When the plurality of flexible sheets 100 are wound to form the cylindrical stent, the side opening 130 will be covered or closed. The side opening 130 of each flexible sheet 100 is adjustable, i.e., as the flexible sheet 100 undergoes expansive or contractive winding in the circumferential direction, the side opening 130 of the flexible sheet 100 can be increased or reduced in angle accordingly. For example, the angle of the side opening 130 of the flexible sheet 100 can be freely adjusted between 0° and 60°, forming the adjustability of the side opening 130. At this time, when there are two or more flexible sheets 100, the two or more flexible sheets 100 can form the cylindrical stent by the interlayer contact of the adjacent flexible sheets 100. Moreover, the two or more side openings 130 respectively formed by the flexible sheets 100 can be evenly distributed along the circumferential direction of the cylindrical stent. The two or more side openings 130 are evenly distributed in the circumferential direction of the cylindrical stent, which enables the cylindrical stent to have uniform circumferential mechanical properties.

Continuing to refer to FIG. 1 and FIG. 4, in some embodiments, the flexible sheet 100 is provided with transition ramps 140 at the ends thereof in the circumferential direction of the cylindrical stent. The transition ramp 140 can be realized by thinning the end portion of the flexible sheet 100 in the circumferential direction. The positions and number of the transition ramps 140 can be determined based on the number and the winding form of the flexible sheets 100, as long as one or more flexible sheets 100 are wound to form the cylindrical stent, and the cylindrical stent can form a smooth surface on its outer wall or inner wall by the transition ramps 140. The smooth surface can reduce the impact on blood flow and vascular stent adhesion.

The flexible sheet 100 can be represented as a sheet material, a film material, etc. The flexible sheet 100 can be made of polymer material. Therefore, the flexible sheet 100 can be a polymer sheet material or a polymer sheet material. For example, in some embodiments, the material of the flexible sheet 100 is a degradable polymer material. The degradable polymer material includes but is not limited to one or more of L-polylactic acid, racemic polylactic acid, etc. Those skilled in the art can choose according to needs, without limitation herein.

Referring to FIG. 10 and FIG. 11, in some embodiments, the restricting member 200 extends along the circumferential direction of the flexible sheet 100, and multiple restricting members 200 are distributed on the flexible sheet 100 along the axial direction of the cylindrical stent. Moreover, a length of the restricting member 200 along the circumferential direction of the cylindrical stent is a first circumferential length, and a length of the flexible sheet 100 along the circumferential direction of the cylindrical stent is a second circumferential length. Therefore, the first circumferential length can be equal to the second circumferential length, or the first circumferential length can be less than the second circumferential length and the multiple restricting members 200 are staggered along the axial direction of the cylindrical stent.

In addition, according to the different requirements of the material, structure, supporting performance and other aspects of the vascular stent, the restricting member 200 can have different shapes, positions, numbers and areas. Those skilled in the art can choose according to needs, without limitation herein.

A preparation method for a vascular stent is provided in the present application. The preparation method for the vascular stent includes the following steps: providing a restricting member 200 on a flexible sheet 100, winding and shaping the flexible sheet 100 into a cylindrical stent, and using the restricting member 200 to restrict the flexible sheet 100 from being wound along a direction that would cause a decrease of the diameter of the cylindrical stent. When winding the flexible sheet 100, the flexible sheet 100 can be wrapped on a folded balloon to form the cylindrical stent, and a shaping process is performed at a suitable temperature. The shape of the cylindrical stent can be fixed by the shaping process, but the shaping process does not involve a pressing and gripping process, i.e., the inner diameter and the outer diameter of the cylindrical stent do not change.

Since the flexible sheet 100 is wrapped on the folded balloon and the folded balloon has not been inflated yet, the cylindrical stent on the folded balloon will have a small diameter. When the cylindrical stent reaches the target position of the blood vessel, the operator can use an automatic expansion of the balloon to apply an external force from the inside of the cylindrical stent to the flexible sheet 100 forming the cylindrical stent, which can cause the flexible sheet 100 to be expansively wound, so that the flexible sheet 100 can increase the diameter of the cylindrical stent by the expansive winding, and the cylindrical stent can be stably attached to an inner wall of the blood vessel.

After the diameter of the cylindrical stent is increased, the cylindrical stent will form a tight fit with the inner wall of the blood vessel. Although the inner wall of the blood vessel will exert a reaction force on the cylindrical stent in an opposite direction at this time, the cylindrical stent will still maintain its present diameter size with the restriction of the restricting member 200, rather than reduce its diameter due to the reaction force exerted by the inner wall of the blood vessel. Obviously, the restricting function of the restricting member 200 enables the cylindrical stent (equivalent to the vascular stent) to have a stronger supporting force, and can maintain a stable and tight fit with the inner wall of the blood vessel in the blood vessel, and can also enable the cylindrical stent (equivalent to the vascular stent) to have higher fatigue resistance.

The technical features of the above embodiments can be randomly combined. To simplify the description, not all possible combinations of the technical features in the above embodiments are described. However, as long as there is no contradiction in the combination of these technical features, all the combinations should be considered to be included within the scope of this specification.

The above-described embodiments only illustrate several embodiments of the present application, and the descriptions of which are relatively specific and detailed, but should not be construed as limiting the scope of the patent application. It should be noted that, for those of ordinary skill in the art, several modifications and improvements can be made without departing from the concept of the present application, and these all fall within the protection scope of the present application. Therefore, the protection scope of the present application should be determined by the appended claims.

## Claims

1. A vascular stent, comprising:
a cylindrical stent, the cylindrical stent comprising at least one flexible sheet, a circumferential direction of the cylindrical stent being defined as a winding direction, the at least one flexible sheet being wound along the winding direction; and
a restricting member provided on the flexible sheet, and configured to restrict the flexible sheet from being wound along a direction that would cause a decrease of the diameter of the cylindrical stent.

2. The vascular stent according to claim 1, wherein the restricting member and the flexible sheet are integrally formed.

3. The vascular stent according to claim 1, wherein the restricting member is fixedly connected to the flexible sheet.

4. The vascular stent according to claim 1, wherein the flexible sheet has overlapping areas, the overlapping areas of the flexible sheet forming interlayer contact within the cylindrical stent, the restricting member being at least partially located in the overlapping areas.

5. The vascular stent according to claim 4, wherein the cylindrical stent is formed by winding a single flexible sheet, the overlapping areas of the single flexible sheet include different parts, and the different parts form interlayer contact within the cylindrical stent.

6. The vascular stent according to claim 4, wherein the cylindrical stent is formed by winding at least two flexible sheets, and the overlapping areas on different flexible sheets form interlayer contact within the cylindrical stent.

7. The vascular stent according to claim 6, wherein each of the flexible sheets is wound to form a cylindrical unit, the cylindrical unit has a side opening, and the at least two flexible sheets are stacked to form the cylindrical stent.

8. The vascular stent according to claim 7, wherein the at least two side openings are evenly distributed along the circumferential direction of the cylindrical stent.

9. The vascular stent according to claim 4, wherein the restricting member comprises unidirectional limiting racks arranged in the overlapping areas, and the unidirectional limiting racks within adjacent overlapping areas which form interlayer contact slide relative to each other or lock each other in two opposite circumferential directions, respectively.

10. The vascular stent according to claim 4, wherein the restricting member comprises unidirectional limiting fluff arranged on the overlapping area, and the unidirectional limiting fluff within adjacent overlapping areas which form interlayer contact generates a first friction force and a second friction force respectively along two opposite circumferential directions, and the first friction force and the second friction force are different.

11. The vascular stent according to claim 1, wherein the cylindrical stent defines a circumferential restricting trajectory around the circumferential direction of the cylindrical stent, and the restricting member restricts the relative slidably winding between adjacent overlapping areas of the flexible sheet that form interlayer contact, along the circumferential restricting trajectory.

12. The vascular stent according to claim 1, wherein the flexible sheet is provided with transition ramps at ends thereof along the circumferential direction of the cylindrical stent, and the cylindrical stent forms a smooth surface through the transition ramps.

13. The vascular stent according to claim 1, wherein the flexible sheet is made of a degradable polymer material.

14. The vascular stent according to claim 1, wherein the restricting member comprises multiple restricting members, and the multiple restricting members are distributed on the flexible sheet along an axial direction of the cylindrical stent.

15. The vascular stent according to claim 14, wherein a length of the restricting member along the circumferential direction of the cylindrical stent is a first circumferential length, a length of the flexible sheet along the circumferential direction of the cylindrical stent is a second circumferential length, and the first circumferential length is equal to the second circumferential length.

16. The vascular stent according to claim 14, wherein a length of the restricting member along the circumferential direction of the cylindrical stent is a first circumferential length, a length of the flexible sheet along the circumferential direction of the cylindrical stent is a second circumferential length, the first circumferential length is smaller than the second circumferential length, and the multiple restricting members are staggered along the axial direction of the cylindrical stent.

17. A preparation method for a vascular stent, comprising:
providing a restricting member on the flexible sheet, winding and shaping the flexible sheet into a cylindrical stent, and using the restricting member to restrict the flexible sheet from being wound along a direction that would cause a decrease of the diameter of the cylindrical stent.
